Europäisches Patentamt

European Patent Office

Office européen des brevets

(11) Veröffentlichungsnummer : **0 066 059**
**B1**

(12)                    EUROPÄISCHE PATENTSCHRIFT

(45) Veröffentlichungstag der Patentschrift :
28.11.84

(21) Anmeldenummer : 82102862.8

(22) Anmeldetag : 03.04.82

(51) Int. Cl.³ : **C 07 C 69/14, C 07 C 67/08**

(54) Verfahren zur Veresterung von Essigsäure mit C2- bis C5-Alkoholen.

(30) Priorität : 29.05.81 DE 3121383

(43) Veröffentlichungstag der Anmeldung :
08.12.82 Patentblatt 82/49

(45) Bekanntmachung des Hinweises auf die Patenterteilung : 28.11.84 Patentblatt 84/48

(84) Benannte Vertragsstaaten :
BE DE FR GB IT NL SE

(56) Entgegenhaltungen :
EP-A- 0 048 987
GB-A- 729 305
Angewandte Chemie 66 (1954)9, Seiten 241,242
Chemische Technik 11)1959)1, Seiten 24-26

(73) Patentinhaber : CHEMISCHE WERKE HÜLS AG
- RSP Patente / PB 15 - Postfach 13 20
D-4370 Marl 1 (DE)

(72) Erfinder : Alfs, Helmut
Bitterfelder Strasse 57
D-4370 Marl (DE)
Erfinder : Böxkes, Werner, Dr.
Oppauer Strasse 2
D-4370 Marl (DE)
Erfinder : Vangermain, Erwin, Dr.
Leverkusener Strasse 1
D-4370 Marl (DE)

## Beschreibung

Es ist bekannt, Essigsäureester technisch durch Veresterung der Essigsäure mit Alkoholen in flüssiger Phase herzustellen. Man kann die Ester zwar auch durch Veresterung in der Gasphase herstellen, dies wird jedoch technisch bisher noch nicht durchgeführt. Weiterhin kann man Ethylacetat nach Tischtschenko in Gegenwart von Aluminiumalkoholat direkt aus Acetaldehyd gewinnen.

Die wichtigen Katalysatoren für die Veresterung von Essigsäure mit Alkoholen in der Flüssigphase sind Brönsted-Säuren, insbesondere Schwefelsäure. Außer Schwefelsäure sind auch Salzsäure, Chlorsulfonsäure und Toluolsulfosäure sowie Gemische von Schwefelsäure mit organischen, beispielsweise p-Toluolsulfosäure, oder schwächeren anorganischen Säuren, beispielsweise Phosphorsäure, geeignet.

Da die niedrig siedenden Essigsäureester mit $C_2$- bis $C_5$-Alkoholen Azeotrope mit Wasser bilden, dosiert man nach den Verfahren der DE-PS 824 341 und der FR-PS 1 068 367 ein äquimolares Gemisch von Essigsäure und Alkohol (Ethanol, Propanol, Butanol) und Katalysator (Schwefelsäure) kontinuierlich in eine Kolonne bzw. Destillationsblase, erhitzt und destilliert den Ester zusammen mit Wasser als azeotropes Gemisch ab, das sich nach der Kondensation in eine Ester- und eine Wasserschicht trennt. Einen kleinen Teil der Ester und/oder der Wasserschicht führt man in die Kolonne zurück, während man den hauptteil des azeotropen Gemisches zur weiteren Aufarbeitung abzieht.

Nach dem Verfahren der GB-PS 729 305 erfolgt die Veresterung in Gegenwart eines Säurekatalysators, .z. B. Schwefelsäure, in einem separaten Reaktor. Das Reaktionsprodukt gibt man anschließend dampfförmig in eine Destillationskolonne. Den Ester destilliert man zusammen mit dem bei der Veresterung anfallenden Wasser ab. Die nicht umgesetzte Carbonsäure führt man aus dem Sumpf der Kolonne flüssig in den Reaktor zurück. Dieses Verfahren erfordert lange Verweilzeiten von 2 bis 5 Stunden.

Veresterungen in Gegenwart von wäßriger Schwefelsäure haben den Nachteil, daß sie in Apparaten aus kostspieligem korrosionsfestem Metall, vorwiegend Emaille, durchgeführt werden müssen. Außerdem muß fortlaufend ein Teil der durch Veresterung verbrauchten Schwefelsäure aus dem Prozeß abgezogen und durch Frisch-Schwefelsäure ersetzt werden. Die abgezogene verbrauchte Schwefelsäure ersetzt werden. Die abgezogene verbrauchte Schwefelsäure muß vor Ablauf in das Fabrikationsabwasser neutralisiert werden und stellt zudem eine Abwasserbelastung dar. Durch den einsatz von Katalysatoren wie p-Toluolsulfosäure kann man die Korrosivität der Reaktionsmischung zwar erniedrigen, die übrigen Nachteile aber nicht eliminieren.

Diese Nachteile lassen sich durch Einsatz von sauren Ionenaustauschern, insbesondere auf Basis von mit Divinylbenzol vernetzten sulfonsauren Vinylpolymeren vermeiden (DE-PS 882 091, Chem. Technik 5, 187 (1953) u. 11, 24, (1959) ; Angew. Chem. 66, 241 (1954)).

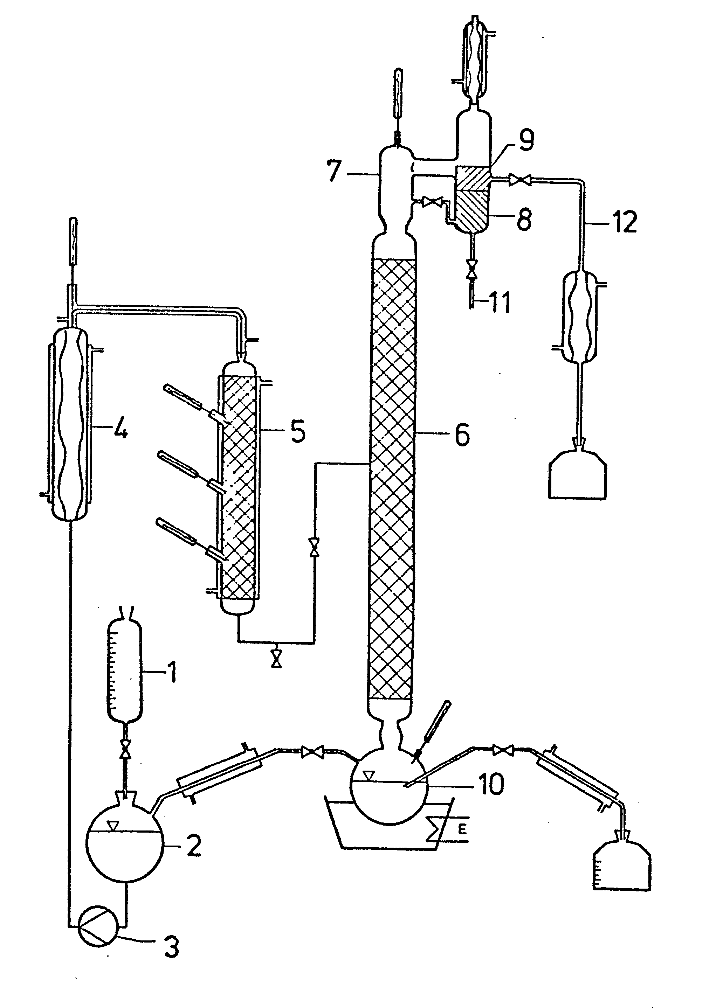

Mit dem Einsatz dieser Ionenaustauscher sind jedoch andere Nachteile verbunden. Bei ihrem Einsatz sind nur verhältnismäßig geringe Durchsätze möglich (Chem. Technik 11 (1959), Seite 24, Tafel 1 und Bild 1). Führt man die Veresterung in eine teilweise mit Ionenaustauschern gefüllten Veresterungskolonne durch (EP-Patentanmeldung 81 10 7710.6, Veröffentlichungsnummer 0 048 987), so erreicht man nur sehr niedrige Raum-Zeit-Ausbeuten. Da. die handelsüblichen, perlförmigen Austauscher mit einem Durchmesser von 0,3 bis 1,5 mm infolge von Flüssigkeitsstau in der Veresterungskolonne zu schwierigkeiten führen, setzt man grobstückige Katalysatoren mit einem Durchmesser von 8 bis 15 mm ein. Diese Grobkatalysatoren, hergestellt durch Beimischung von thermoplastischen Massen während der Polymerisation von Styrol-Divinylbenzol, sind jedoch in der Aktivität gegenüber den feinkörnigen Perlkatalysatoren stark abgeschwächt. Zudem haben die grobstückigen Katalysatoren infolge ihrer geringen mechanischen Festigkeit einen erheblichen Abrieb.

Daraus ergibt sich die Aufgabe, ein Verfahren zu finden, das diese Nachteile vermeidet und die Herstellung von Essigsäureestern mit $C_2$- bis $C_5$-Alkoholen mit einem hohen Reinheitsgrad in einfacher und wirtschaftlicher Weise in guter Ausbeute ermöglicht.

Diese Aufgabe wird erfindungsgemäß entsprechend den Angaben der Ansprüche gelöst.

Das erfindungsgemäße Verfahren eignet sich für die Veresterung von Essigsäure mit Ethanol, Propanol, iso-Propanol, n-, iso-, und tert.-Butanol sowie mit den Amylalkoholen, bevorzugt für die Veresterung von Essigsäure mit iso- und n-Butanol.

Die Veresterung führt man bei einer Temperatur von 70 bis 140 °C durch, vorzugsweise bei einer Temperatur von 90 bis 120 °C, bei Normaldruck, Unter- oder Überdruck. Im Unterdurckbereich arbeitet man bei 0,2 bis 0,99 bar, im Überdruckbereich bei 1,01 bis 10 bar. Im Überdruckbereich regelt man vorzugsweise den Druck im Reaktor mit Hilfe eines Druck-Überstromventils, so daß der Katalysator mit der Reaktionsmischung geflutet ist.

Als Katalysatoren sind grundsätzlich alle starke sauren Kationenaustauscher geeignet. Sie können sowohl gelförmig als auch makroporös sein. Im allgemeinen setzt man handelsübliche Ionenaustauscher mit einem Kornspektrum von 0,4 bis 1,3 mm ein. Man kann jedoch auch pulver- oder granulatförmige Ionenausstauscher als Veresterungskatalysatoren einsetzen. Ein Teil der $H^+$ Ionen der sauren Kationenaustauscher kann durch Metalle ersetzt sein. Die Katalysatormenge ist vorteilhaft so zu bemessen, daß das ablaufende Gemisch sich ins Gleichgewicht gesetzt hat, entsprechend einem Durchsatz von 1 bis 20 g des Einsatzgemisches/h/g trockenen Kontakt. Vor dem Einsatz quillt man den Kationenaustauscher in dem umzusetzenden Alkohol bis zur Volumenkonstanz.

Das Molverhältnis Alkohol zu Essigsäure beträgt im allgemeinen 1 : 1 bis 10 : 1, vorzugsweise 1 : 1 bis 2 : 1. Als Veresterungsreaktoren eignen sich alle Reaktortypen, die als Festbettreaktoren üblicherweise eingesetzt werden, wie Schachtofen oder Rohrbündelreaktoren, vorteilhafterweise ein Schachtofen, der am unteren Ende vor der Verjüngung einen Flansch besitzt. Zwischen dem Flansch befindet sich auf einer Lochblechplatte ein feinmaschiges Sieb mit einer Maschenweite von < 0,5 mm.

Die Veresterung erfolgt beispielsweise in der Weise (s. Abb.), daß man eine Mischung aus Essigsäure und einem $C_2$- bis $C_5$-Alkohol, z. B. Butanol, aus einer Vorlage (2) mit einer Dosierpumpe (3) in einen Wärmetauscher (4) fördert und dort auf die Reaktionstemperatur, beispielsweise 90 bis 120 °C, aufheizt. Diese aufgeheizte Mischung gibt man oben in einen Reaktor (5), in dem sich der stark saure Kationenaustauscher, der zuvor mit dem Alkohol bis zur Volumenkonstanz gequollen ist, befindet. Die Reaktionsmischung durchströmt den Reaktor von oben nach unten bzw. von unten nach oben. Der Reaktor wird durch einen Wärmeträger auf Reaktionstemperatur gehalten.

Im Reaktor erfolgt die Veresterung bis zum Gleichgewicht. Das aus dem Reaktor ausströmende Veresterungsgemisch fährt man in die untere Hälfte einer nachgeschalteten Destillationskolonne (6). Den Sumpf der Kolonne heizt man auf eine Temperatur von 85 bis 120 °C und destilliert den gebildeten Ester mit dem Reaktionswasser und geringen Mengen Alkohol als azeotropes Gemisch über Kopf der Kolonne ab. Dieses azeotrope Gemisch trennt man gegebenenfalls in einem Phasenscheider (7) in eine wäßrige (8) und eine organische Phase (9). Über den Syphon (8) fährt man gegebenenfalls einen Teil des Wassers und am Überlauf (9) gegebenenfalls einen Teil der organischen Phase auf den Kopf der Kolonne zurück. Diese Teil-Rückführung des Wassers ist gegebenenfalls erforderlich, um das azeotrope Wasser-Ester-Verhältnis in der Kolonne zu halten. Diese Fahrweise gewährleistet gleichzeitig die Säurefreiheit im Rohester. Das Reaktionswasser wird gegebenenfalls im äquimolaren Verhältnis zum Esters aus dem Abscheider (11) kontinuierlich abgezogen, gegebenenfalls fährt man auch einen Teil des Esters auf den Kopf der Kolonne zurück, wobei man das Rücklaufverhältnis so einstellt, daß die Übergangstemperatur des azeotropen Gemisches gehalten wird, beispielsweise bei Isobutylacetat bei 88 °C.

Den entstandenen Roh-Ester zieht man im Seitenabzug des Abscheiders (12) zur Reindestillation ab. Die nicht umgesetzten Einsatzstoffe Alkohol und/oder Essigsäure sammeln sich im Sumpf (10) der Kolonne an, aus dem man sie kontinuierlich dampfförmig in der Vorlage (2) abzieht und nach Ergänzung der Mengen entsprechend dem aus dem Prozeß abgezogenen Ester mit dem Alkohol-Essigsäure-Gemisch aus der Vorlage (1) wieder dem Reaktor zuführt. Die gegebenenfalls bei der Veresterung anfallenden, hochsiedenden Verunreinigungen sammeln sich als Destillationsrückstände im Sumpf (10) der Kolonne an und können von dort günstig ausgeschleust werden.

Bei dampfförmigem Abzug der nicht umgesetzten Komponenten Alkohol und Essigsäure bleiben die Hochsieder im Sumpf und werden flüssig aus dem Sumpf abgezogen.

Obwohl nach den Angaben in Chem. Technik 11 (1959) Seite 24, Tafel 1 und Bild 1 die Veresterung an Ionenaustauschern erheblich langsamer verläuft als mit $H_2SO_4$, sind beim erfindungsgemäßen Verfahren überraschenderweise sogar wesentlich kürzere Verweilzeiten erforderlich als beim Verfahren der GB-PS 729 305 mit Schwefelsäure als Katalysator. Man benötigt nur 1/20 bis 1/50 der Verweilzeit des Verfahrens der GB-PS 729 305.

Bei der nachfolgenden Reinestergewinnung gibt man das abgezogene Gemisch (12), Ester, Alkohol und geringe Mengen Wasser, mittig in eine Kolonne und zieht über Kopf den gesamten Alkohol, das gesamte Wasser und möglichst geringe Mengen Ester ab. Den Kopfabzug fährt man, gegebenenfalls unter Abtrennen des Wassers, wieder in den Veresterungsreaktor zurück. Der wasser- und alkoholfreie Ester fällt im Sumpf der Kolonne an. Je nach dem geforderten Reinheitsgrad zieht man ihn im Seitenstrom an den untersten Böden dampfförmig ab oder fährt ihn flüssig aus dem Sumpf in eine zweite Kolonne ein, in der der Reinester dann als Kopfprodukt anfällt.

Die erhaltenen Essigsäureester verwendet man als Lösemittel für Nitrocellulose, Celluloid, Alkylharze, Vinylharze, Chlorkautschuk, Polystyrol und als Lacklösemittel.

Beispiel 1

Isobutylacetat (Abb.)

In ein ummanteltes Rohr (5) (Ø 35 mm, Höhe 65 cm) werden 150 g trockener, bis zur Volumenkonstanz mit Isobutanol gequollener, stark saurer Kationenaustauscher (Lewatit SPC 118 = Polystyrolsulfonsäure mit 18 Gew.% Divinylbenzol vernetzt, Korngröße 0,3 bis 1,3 mm) eingefüllt. Das Volumen des gequollenen Katalysators beträgt 480 cm³. Das Katalysator-Festbett ruht auf einem VA Maschengewebe von 0,3 mm Maschenabstand, so daß ein Austragen des Katalysators während der Veresterung aus dem Reaktor verhindert wird. 780 g/h eines Gemisches Isobutanol-Essigsäure im Molverhältnis 1,2 : 1 wird aus der Vorlage (1) mittels Dosierpumpe (3) durch den Vorwärmer (4) gepumpt und auf 115 °C aufgeheizt.

Aus dem Vorwärmer durchströmt das Gemisch von oben nach unten den Reaktor (5), wobei die Veresterung bis zum Gleichgewicht erfolgt (ca. 68 %). Die Reaktionstemperatur im Reaktor beträgt 115 °C. Die Temperatursteuerung erfolgt durch Umpumpen von thermostatisch gesteuertem Glykol durch die Heizmäntel des Vorwärmers und des Reaktors. Das den Reaktor verlassende Veresterungsgemisch wird in die untere Hälfte der dem Reaktor nachgeschalteten Füllkörper-Kolonne (6) (Ø 50 mm, Länge 180 cm, Füllkörper : 6 mm Ringe) eingefahren, deren Sumpftemperatur 110 °C beträgt. Hierbei destilliert bei 88 °C der gebildete Ester, geringe Mengen Alkohol und das gesamte Reaktionswasser über Kopf der Kolonne ab. Das Destillat wird im Phasenabscheider (7) in eine wäßrige (8) und organische Phase (9) getrennt. Über den Syphon (8) wird ein Teil des Wassers und am Überlauf (9) ein Teil der organischen Phase auf den Kopf der Kolonne zurückgefahren. Das Rücklaufverhältnis der organischen Phase, bezogen auf den gebildeten Ester, beträgt 2, das Rücklaufverhältnis der wäßrigen Phase, bezogen auf das Reaktionswasser, beträgt 4.

Das Reaktionswasser wird im äquimolaren Verhältnis zum Ester aus dem Abscheider (11) kontinuierlich ausgeschleust, und der gebildete Ester (mit geringen Mengen Alkohol und Wasser) im Seitenabzug des Abscheiders (12) aus dem Veresterungsprozeß abgezogen. Die nicht umgesetzten Einsatzstoffe sammeln sich im Sumpf (10) der Kolonne an und werden kontinuierlich, dampfförmig aus der Blase in die Vorlage (2) geführt und nach Ergänzung (Mengen entsprechend dem aus dem Prozeß abgezogenen Ester) mit dem Alkohol-Säure-Gemisch aus Vorlage 1 wieder dem Reaktor zugeführt.

Die während der Veresterung anfallenden hochsiedenden Verunreinigungen fallen bei diesem Prozeß als Destillationsrückstände in der Blase der Kolonne (6) an und werden kontinuierlich aus der Flüssigphase aus dem Prozeß ausgeschleust.

Nach 52 Stunden Laufzeit erhält man 36,2 kg Rohester mit folgender Zusammensetzung (GC-Analyse) :

| | |
|---|---|
| $H_2O$ | = 2,1 Gew.% |
| Essigsäure | = 0,0 Gew.% |
| iso-Butylalkohol | = 13,1 Gew.% |
| iso-Butylacetat | = 84,8 Gew.% |
| | = 0,59 kg/h entspr. 3,94 kg Ester/kg Katalysator/h |

Die Ausbeute an iso-Butylacetat bezogen auf die umgesetzten Komponenten Alkohol-Essigsäure beträgt unter Berücksichtung der Apparatefüllung > 99 Gew.% d. Th.

## Beispiel 2

### Ethylacetat

600 g/h einer Mischung Ethanol-Essigsäure im Verhältnis 1,1 Mol Ethanol : 1,0 Mol Essigsäure setzt man nach den Angaben des Beispiels 1 um. Bei einer Temperatur von 93 °C im Reaktor destilliert am Kopf der Kolonne (Sumpftemperatur : 98 °C) bei einem Rücklaufverhältnis von 2 und bei einer Temperatur von 72 °C ein homogenes Gemisch, bestehend aus Ethanol, Wasser und Ethylacetat über. Der Durchsatz beträgt 0,6 kg Mischung/h entsprechend 0,46 kg Ester.

Der anfallende Rohester hat gaschromatographisch bestimmt folgende Zusammensetzung :

| | |
|---|---|
| Wasser | = 6,0 Gew.% |
| Essigsäure | = 0,0 Gew.% |
| Ethanol | = 6,1 Gew.% |
| Ethylacetat | = 88,9 Gew.% |

Die Esterausbeute, bezogen auf den Säure-Alkoholumsatz, beträgt unter Berücksichtigung der Apparatefüllung > 98 Gew.% der Th. Setzt man statt des Rücklaufverhältnisses von 2 ein Rücklaufverhältnis von 20 ein, so erhält man ein Kopfprodukt, das nach der Säurezahl frei von Essigsäure ist.

## Beispiel 3

### Isopropylacetat

800 g/h einer Mischung von iso-Propanol-Essigsäure im Molverhältnis 1,2 Mol iso-Propanol : 1 Mol Essigsäure setzt man nach den Angaben des Beispiels 1 um. Die Temperatur im Reaktor beträgt 96 °C. Am Kopf der Kolonne (Sumpftemperatur : 112 °C) destilliert bei einer Temperatur von 76 bis 77 °C ein Gemisch von Ester/Wasser über und trennt sich in eine wäßrige und organische Phase. Einen Teil der organischen Phase fährt man auf den Kopf der Kolonne zurück (Rücklaufverhältnis 3 : 1), während das gesamte anfallende Wassers abgezogen wird.

Der anfallende Rohester hat gaschromatographisch bestimmt folgende Zusammensetzung :

| | |
|---|---|
| Wasser | = 8,1 Gew.% |
| Essigsäure | = 0,0 Gew.% |
| Isopropanol | = 11,1 Gew.% |
| Isopropylacetat | = 80,8 Gew.% |

Die Ausbeute, bezogen auf den Säure-Alkoholumsatz, beträgt unter Berücksichtigung der Apparatefüllung > 98 Gew.%.

Beispiel 4

n-Butylacetat

1 340 g/h einer Mischung von n-Butanol-Essigsäure im Verhältnis 1,2 Mol n-Butanol : 1,0 Mol Essigsäure werden nach den Angaben des Beispiels 1 umgesetzt. Am Kopf der Kolonne (Sumpftemperatur 115 °C) destilliert bei einer Temperatur von 89 °C das n-Butylacetat/Wassergemisch über. Das Gemisch trennt sich in eine wäßrige und eine organische Phase. Ein Teil der wäßrigen und organischen Phase fährt man auf den Kopf der Kolonne zurück (2 Teile organische Phase und 4 Teile Wasser).

Es werden pro Stunde 1,2 kg Rohester = 6,9 kg/kg Katalysator mit folgender Zusammensetzung am Kopf der Kolonne abgezogen (gaschromatographisch bestimmt) :

| Wasser | = 2,0 Gew.% |
| Essigsäure | = 0,0 Gew.% |
| n-Butanol | = 11,6 Gew.% |
| n-Butylacetat | = 86,4 Gew.% |

Die Esterausbeute, bezogen auf den Säure-Alkoholeinsatz, beträgt > 99,0 Gew.% d. Th.

## Ansprüche

1. Verfahren zur Veresterung von Essigsäure mit $C_2$- bis $C_5$-Alkoholen in Gegenwart eines Säurekatalysators, wobei man die Veresterung in einem vertikalen Reaktor durchführt, in Verbindung mit einer Destillationskolonne, in die man das den Reaktor verlassende Reaktionsgsgemisch einfährt, den gebildeten Ester und das Wasser als azeotropes Gemisch über Kopf der Kolonne abzieht und das azeotrope Gemisch gegebenenfalls in eine organische und eine wäßrige Phase trennt, dadurch gekennzeichnet, daß man als Katalysator einen stark sauren Ionenaustauscher einsetzt und das Rücklaufverhältnis des azeotropen Gemisches auf 0,5 bis 20, bzw. das der organischen Phase, bezogen auf die gebildete Menge des Esters, auf 0 bis 20 und das der wäßrigen Phase, bezogen auf die gebildete Menge Wasser auf 0 bis 20 einstellt und man aus dem Sumpf die nicht umgesetzte Essigsäure sowie den nicht umgesetzten $C_2$- bis $C_5$-Alkohol kontinuierlich dampfförmig abzieht und mit der entsprechenden Menge des über Kopf abgezogenen Esters an Essigsäure sowie an $C_2$- bis $C_5$-Alkohol ergänzt wieder in den Veresterungsreaktor eingibt.

2. Verfahren nach Anspruch 1, dadurch gekennzeichnet, daß das Rücklaufverhältnis des azeotropen Gemisches bzw. der organischen Phase, bezogen auf die Menge des Esters, 1 bis 4 und das Rücklaufverhältnis der wäßrigen Phase, bezogen auf die gebildete Menge Wasser, 2 bis 6 beträgt.

## Claims

1. Process for the esterification of acetic acid with $C_2$-$C_5$-alcohols in the presence of an acid catalyst, wherein the esterification is carried out in a vertical reactor in conjunction with a distillation column into which the reaction mixture leaving the reactor is introduced, the ester formed, and the water, being taken off as an azeotropic mixture via the top of the column and the azeotropic mixture being separated, if appropriate, into an organic phase and an aqueous phase, characterised in that the catalyst used is a strongly acidic ion exchanger and that the reflux ratio of the azeotropic mixture is set to 0.5-20 or the reflux ratio of the organic phase is set to 0-20, based on the amount of ester formed, and the reflux ratio of the aqueous phase is set to 0-20, based on the amount of water formed, and that the unconverted acetic acid and the unconverted $C_2$-$C_5$-alcohol are continuously taken off, as vapour, from the bottom and are reintroduced into the esterification reactor together with supplementary amounts of acetic acid and of $C_2$-$C_5$-alcohol corresponding to the amount of ester taken off at the top.

2. Process according to Claim 1, characterised in that the reflux ratio of the azeotropic mixture or of the organic phase, based on the amount of ester, is 1-4 and the reflux ratio of the aqueous phase, based on the amount of water formed, is 2-6.

## Revendications

1. Procédé pour l'estérification de l'acide acétique avec des alcools en $C_2$ à $C_5$, en présence d'un catalyseur acide, tandis qu'on effectue l'estérification dans un réacteur vertical, en liaison avec une colonne de distillation, dans laquelle on introduit le mélange réactionnel quittant le réacteur, que l'on soutire à la tête de la colonne, sous forme d'un mélange azéotropique, l'ester formé et l'eau, puis que l'on sépare éventuellement ce mélange azéotropique en une phase organique et en une phase aqueuse, caractérisé par le fait qu'on utilise, comme catalyseur, un échangeur d'ions fortement acide et que l'on règle à une valeur de 0,5 à 20 le rapport de reflux du mélange azéotropique ou que l'on ajuste celui de la phase organique, relativement à la quantité de l'ester qui s'est formée, à une valeur de 0 à 20, et que l'on règle celui de la phase aqueuse à une valeur de 0 à 20, relativement à la quantité d'eau formée, et que l'on soutire en continu à l'état de vapeur, du produit de pied de la colonne, l'acide acétique non converti, ainsi que l'alcool en $C_2$ à $C_5$ qui n'a pas réagi, et qu'après avoir complété le mélange soutiré avec la quantité d'acide acétique, ainsi que d'alcool en $C_2$ à $C_5$, qui correspond à la quantité de l'ester soutiré à la tête de la colonne, on introduit à nouveau le tout dans le réacteur d'estérification.

2. Procédé selon la revendication 1, caractérisé par le fait que le rapport de reflux du mélange azéotropique ou de la phase organique, relativement à la quantité de l'ester, est de 1 à 4 et que le rapport de reflux de la phase aqueuse, relativement à l'eau formée, est de 2 à 6.